# EUROPEAN PATENT APPLICATION

(11) **EP 3 575 788 A1**
(43) Date of publication of application: **04.12.2019**
(21) Application number: 18744017.7
(22) Date of filing: 26.01.2018
(51) Int. Cl.: G01N 33/48

(54) **MONONUCLEAR CELL SEPARATING DEVICE AND MONONUCLEAR CELL SEPARATING METHOD**

(30) Priority: 27.01.2017 JP 2017012742
(71) Applicant: Foundation for Biomedical Research and Innovation at Kobe, Kobe-shi Hyogo 650-0047 (JP); JTEC Corporation, Ibaraki-shi Osaka 567-0086 (JP)
(72) Inventor: TAGUCHI, Akihiko, Kobe-shi Hyogo 650-0047 (JP); NIINO, Yukiko, Kobe-shi Hyogo 650-0047 (JP); TSUMURA, Takashi, Kobe-shi Hyogo 650-0047 (JP); MORITA, Kenichi, Kobe-shi Hyogo 650-0047 (JP)
(74) Representative: J A Kemp LLP
(86) International application number: PCT/JP2018/002497
(87) International publication number: WO 2018/139583

(57) **Abstract**

The mononuclear cell separation apparatus of the present invention has an injecting means (210) for injecting a centrifugation medium from the bottom surface of a container (100) storing a blood sample; a centrifugation means (300) for centrifuging a container (100) containing a centrifugation medium and a blood sample layered in this order from the bottom surface side; a detecting means (400) for detecting a clot present at a mononuclear cell layer after centrifugation; a removing means (220) for removing a detected clot; and a harvesting means (230) for harvesting the mononuclear cell. The mononuclear cell separation method of the present invention includes an injecting step, a centrifuging step, a detection step, a removing step, and a harvesting step corresponding to each constituent element of the mononuclear cell separation apparatus of the present invention.

## Description

### [Technical Field]

The present invention relates to a mononuclear cell separation apparatus and a method for separating a mononuclear cell.

### [Background Art]

In recent years, a cell therapy for promoting regeneration of blood vessels has been initiated which includes collecting bone marrow fluid of ischemic disease patient, separating mononuclear cells present in the bone marrow fluid by a specific gravity centrifugation method, and transplanting the separated mononuclear cells to a local site of ischemia or in the blood vessels. The purpose of separating mononuclear cells from the bone marrow fluid using a specific gravity centrifugation method is to remove granulocytes and erythrocytes contained in the bone marrow fluid, and removal of these cells is considered to improve the therapeutic effect of cell therapy.

On the other hand, since bone marrow fluid is coagulated extremely easily, it is very difficult to completely prevent formation of clot even when anticoagulants are sufficiently used during collection of bone marrow fluid from patients. A clot is formed by erythrocyte entangled on a network structure formed by the polymerization of fibrin, and the main components of the clot are fibrin, platelet and erythrocyte. In treatments using bone marrow cells, clots often interfere with the treatment. Therefore, at the time of allogeneic bone marrow cell transplantation in leukemia patients, for example, 500 ml or more of bone marrow fluid is collected, clots are removed by passage through three kinds of bone marrow transplantation filters with different pore sizes, and mononuclear cells present in the bone marrow fluid are separated by a specific gravity centrifugation method. Regenerative therapy using autologous bone marrow fluid in patients with limb ischemia has been shown to promote revascularization (non-patent document 1). Similar to allogeneic bone marrow cell transplantation for leukemia patients, 500 ml or more of bone marrow fluid is collected, clots are removed by passage through three kinds of bone marrow transplantation filters with different pore sizes, mononuclear cells present in the bone marrow fluid are separated by a specific gravity centrifugation method and using a cell separation apparatus, and the cells are transplanted to the patients. In addition, regenerative therapy using autologous bone marrow fluid in patients with cerebral infarction has been shown to promote recovery of nerve function (non-patent document 2). 25 ml or 50 ml of bone marrow fluid is collected, which is smaller in amount than for leukemia patients and limb ischemia patients, mononuclear cells are separated manually in a cell processing center, clots contaminating the layer containing the mononuclear cells are manually removed, and the cells are transplanted to the patients. The reasons for using a relatively small amount of bone marrow fluid in patients with cerebral infarction compared to patients with limb ischemia are that a sufficient effect can be expected even with a small amount (non-patent document 3) and that a decreased blood pressure due to collection of a large amount of bone marrow may exacerbate the symptoms of cerebral infarction. In addition, when clots are removed using a filter during separation and purification of a relatively small amount of bone marrow fluid, a volume loss in the filter part is large and the volume of the bone marrow fluid decreases significantly. Thus, use of a filter is difficult. Similarly, in a test including manual separation of bone marrow mononuclear cells and administration to myocardial infarction patients, the effectiveness of cell transplantation has been shown (non-patent document 4). However, the effectiveness was not acknowledged in a clinical trial using cells separated by cell separation equipment without a clot removal function (non-patent document 5). Manual separation of mononuclear cells in a cell processing center requires complicated operations and enormous expense for building and maintaining the cell processing center. Therefore, a bone marrow mononuclear cell separation apparatus that does not require a cell processing center has been invented besides existing instruments, as in patent document 1. However, the device does not have a clot remove function.

### [Document List]

### [Patent document]

patent document 1: WO 2011/001936

### [non-patent documents]

non-patent document 1: Taguchi et al. Eur J Vasc Endovasc Surg. 2003 Mar; 25(3):276-8
non-patent document 2: Taguchi et al. Stem Cells Dev. 2015 Oct 1; 24(19):2207-18
non-patent document 3: Uemura et al. Curr Vasc Pharmacol. 2012 May; 10(3):285-8.
non-patent document 4: Schachinger V N Engl J Med. 2006 Sep 21; 355 (12) : 1210-21
non-patent document 5: Traverse JH, JAMA. 2012 Dec 12; 308 (22) : 2380-9.

### [SUMMARY OF THE INVENTION]

### [Problems to be Solved by the Invention]

The present invention has been made in view of such problems and aims to provide a mononuclear cell separation apparatus and a mononuclear cell separation method that resist occurrence of contamination of the mononuclear cell component to be harvested with clot-derived cells including erythrocyte and the like.

### [Means of Solving the Problems]

The mononuclear cell separation apparatus of the present invention has an injecting means for injecting a centrifugation medium from the bottom surface of a container storing a blood sample, a centrifugation means for centrifuging a container containing a centrifugation medium and a blood sample layered in this order from the bottom surface side, a detecting means for detecting a clot present at a bone marrow mononuclear cell layer after centrifugation, a removing means for removing a detected clot, and a harvesting means for harvesting a bone marrow mononuclear cell.

The mononuclear cell separation method of the present invention includes an injecting step for injecting a centrifugation medium to the bottom surface of a container storing a blood sample, a centrifugation step for centrifuging a container containing a centrifugation medium and a blood sample layered in this order from the bottom surface side, a detecting step for detecting a clot present in a mononuclear cell layer after centrifugation, a removing step for removing a detected clot, and a harvesting step for harvesting a mononuclear cell.

### [Effect of the Invention]

According to the present invention, contamination of a separated and collected mononuclear cell component with a clot-derived cell including erythrocyte and the like does not occur easily.

### [Brief Description of the Drawings]

Fig. 1 shows an external view of the mononuclear cell separation apparatus of the present embodiment.
Fig. 2 is a conceptual drawing to explain a clot imaging means, in which (A) explains a side imaging means and (B) explains an upper imaging means.
Fig. 3 explains a step from storing a blood sample in a container to moving a nozzle opening end to a container bottom part.
Fig. 4 explains a step from the start of injection of a centrifugation medium into a container bottom part to the completion of centrifugation.
Fig. 5 explains a step from the start of plasma suction to the completion of suction.
Fig. 6 explains a step from the start of removal of a clot present above a mononuclear cell layer to the completion of removal.
Fig. 7 explains a step from the start of harvesting a mononuclear cell layer to the completion of harvesting.
Fig. 8 explains a measurement method of atrophy index.
Fig. 9 shows an ischemic injury suppressive effect of a bone marrow mononuclear cell separated by the method of the present Example.
Fig. 10 shows a cerebral cortex functional recovery promoting effect by the administration of a bone marrow mononuclear cell separated by the method of the present Example.

### [Description of Embodiments]

The embodiment of the present invention is specifically explained in the following by referring to the attached Figures. The embodiment is intended to facilitate understanding of the principle of the present invention. The scope of the present invention is not limited to the following embodiment and other embodiments in which those skilled in the have appropriately substituted the configurations of the following embodiments are also included in the scope of the present invention.

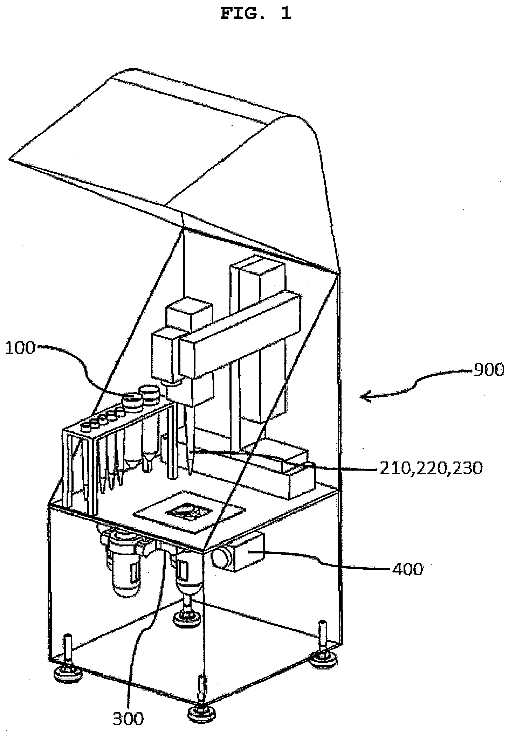

As shown in Fig. 1, the mononuclear cell separation apparatus 900 of the present embodiment has an injecting means 210 for injecting a centrifugation medium from the bottom surface of a container 100 storing a blood sample, a centrifugation means 300 for centrifuging a container 100 containing a centrifugation medium and a blood sample layered in this order from the bottom surface side, a detecting means 400 for detecting a clot present at a mononuclear cell layer after centrifugation, a removing means 220 for removing a detected clot, and a harvesting means 230 for harvesting a mononuclear cell.

The container 100 is, for example, a centrifuge tube made of glass or plastics. The blood sample is not particularly limited and is, for example, a bone marrow fluid. The bone marrow fluid can be collected by, for example, topically or systemically anesthetizing vertebrate (including human), puncturing a needle into a bone, and sucking the fluid with a syringe. Examples of the bone include, but are not limited to, femur, sternum, ilium forming pelvis and the like.

The injecting means 210 for injecting a centrifugation medium is provided with a nozzle capable of injecting a centrifugation medium into the container 100 and connected to a centrifugation medium supply mechanism (not shown), and a moving mechanism (not shown) that moves an opening end of the nozzle to the bottom surface of the container 100 storing the blood sample. As the centrifugation medium supply mechanism, a known liquid supply mechanism can be adopted and is configured with, for example, a tank containing the centrifugation medium, a feed syringe, a flow path, an electromagnetic valve and the like. The centrifugation medium is not particularly limited and, for example, a medium commercially available for forming density gradient such as Percoll (registered trademark), Lymphoprep (registered trademark) and the like can be used. Also, Ficoll-Paque (registered trademark), Nycoprep (registered trademark) and the like can also be used, with preference given to Ficoll-Paque PREMIUM (Ficoll is a registered trademark).

When a centrifugation medium is rapidly injected from the injecting means 210, the interface between the centrifugation medium and the blood sample may not be formed because of the mixture of the centrifugation medium and the blood sample. Thus, injection at a given velocity or below is desirable. Specifically, when the container 100 is a 50 mL centrifuge tube, a centrifugation medium can be injected at 0.05 mL/sec - 1.0 mL/sec.

As the centrifugation means 300, a known centrifugation mechanism can be used. It centrifuges the container 100 containing a centrifugation medium and a blood sample layered in this order from the bottom surface side. The centrifugation means 300 is provided with, for example, 4 buckets formed on a rotating shaft rotated by a motor, and the container 100 is set in the bucket.

When centrifugation is performed, a counter weight to be contained at a position opposite to the container 100 storing blood samples can be prepared by injecting PBS or a blood sample and a centrifugation medium into another container 100 in the above-mentioned injection means 210.

After centrifugation, an erythrocyte layer, a centrifugation medium layer, a mononuclear cell layer, and a plasma layer are formed in this order from the container bottom part in the container 100. The detecting means 400 detects a clot present at the mononuclear cell layer and to be the removal target. As used herein, the clot present at the mononuclear cell layer and to be the removal target encompasses the clot present in the mononuclear cell layer and the clot present above the mononuclear cell layer.

The detecting means 400 is provided with an upper imaging means 410 that images the mononuclear cell layer in the container 100 from the vertically upper direction, a side imaging means 420 that images the mononuclear cell layer in the container 100 from the horizontal direction, and a location information detecting means (not shown) that detects location information of the clot present at the mononuclear cell layer based on the color information obtained from the images taken by the upper imaging means and the images taken by the side imaging means. The upper imaging means 410 and the side imaging means 420 are, for example, CCD cameras, and the images imaged by these CCD cameras are displayed as color images on a monitor (not shown).

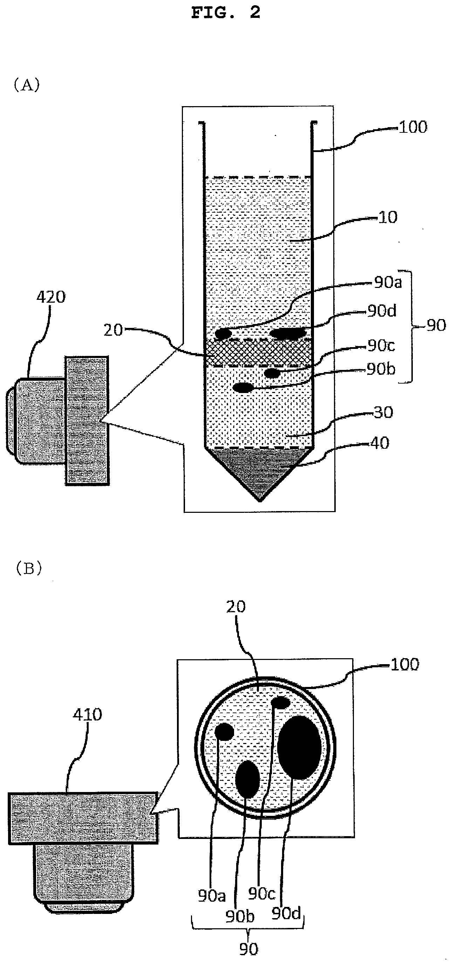

As shown in Fig. 2(A), after centrifugation, the erythrocyte layer 40 is the lowest layer, and the mononuclear cell layer 20 is observed as an approximately white band-like layer between the plasma layer 10 and the centrifugation medium 30. The clot 90 (in Fig. 2, clot 90 is composed of, for example, 90a, 90b, 90c, 90d) is mainly observed as red foreign object because it is mainly composed of erythrocytes.

As shown in Fig. 2(B), clot 90a and 90d with the mononuclear cell layer 20 (white) as the background, clot 90b and 90c positioned below the mononuclear cell layer 20, and a clot (not shown) in the mononuclear cell layer are imaged by the upper imaging means 410. The location information detecting means applies a spatial filter and the like to the image taken by the upper imaging means 410 to emphasize the part where the luminance changes and, for example, binarizes the emphasized part, whereby the location information in the planar view of the clot 90a and 90d with the mononuclear cell layer 20 (white) as the background, the clot 90b and 90c positioned below the mononuclear cell layer 20, and the clot in the mononuclear cell layer is detected.

With only the images taken by the upper imaging means 410, however, it is difficult to distinguish the clot 90a and 90d positioned above the mononuclear cell layer 20, the clot 90b and 90c positioned below the mononuclear cell layer 20, and the clot in the mononuclear cell layer.

In the invention of this embodiment, as shown in Fig. 2(A), the clot 90 is also imaged from the horizontal direction by the side imaging means 420, and the location information detecting means distinguishes the clot 90a and 90d positioned above the mononuclear cell layer 20, the clot 90b and 90c positioned below the mononuclear cell layer 20, and the clot in the mononuclear cell layer based on the images taken by the side imaging means 420. As a result, the location information detecting means can detect the clot present in the mononuclear cell layer 20 and to be the removal target (in Fig. 2, clot 90a and 90d positioned above mononuclear cell layer 20).

From the aspect of work efficiency, it is also possible to detect and remove only a clot of a predetermined size among the clots present above the mononuclear cell layer 20, instead of detecting and removing all the clots present above the mononuclear cell layer 20. For example, when the detecting means 400 regards the clot to have a shape approximating a spheroid, a clot having either an equatorial radius or a polar radius of not less than 5 mm is detected as a clot to be the removal target. For example, the detecting means 400 can calculate the area in plan view of the clots taken by the upper imaging means 410 among the clots present above the mononuclear cell layer 20, and detect one having not less than a given area to be a removal target.

The removing means 220 removes the clot present in the mononuclear cell layer 20 and to be the removal target (in Fig. 2, clot 90a and 90d positioned above the mononuclear cell layer 20), which was detected by the location information detecting means. The removing means 220 is provided with a nozzle connected to a clot suction mechanism (not shown) and capable of sucking a clot, and a moving mechanism (not shown) that moves an opening end of the nozzle to a clot present in the mononuclear cell layer 20.

To facilitate removal of the detected clot, it is possible to provide a plasma layer removal means for removing the plasma layer 10 before removing the clot. As the plasma layer removing means, a known liquid suction mechanism can be adopted, which is configured from, for example, a suction nozzle, a tank for storing sucked plasma layer, a suction pump, a flow path and the like. It is preferable to complete the suction while leaving a small amount of plasma layer 10 for the reason described below, and specifically, it is preferable to complete the suction while leaving 10% - 20% of the liquid amount of the plasma layer 10 in the container 100. The present invention is not limited to the configuration for removing the clot after removing the plasma layer, and it is also possible to remove the clot simultaneously with the removal of the plasma layer by suction, and it is also possible to remove the clot without removing the plasma layer 10.

The harvesting means 230 is provided with a nozzle connected to a mononuclear cell harvesting bag (not shown) and capable of harvesting a mononuclear cell, and a moving mechanism that moves an opening end of the nozzle to the mononuclear cell layer 20. The harvested mononuclear cells can be used as they are, and it is also possible to add, for example, saline before use and wash the cells plural times by a centrifugal separator. When suction of the plasma layer is completed leaving a small amount of plasma layer 10, the remaining plasma layer may also be sucked when harvesting the mononuclear cell layer. Washing the mononuclear cells before use can eliminate contamination of the remaining plasma layer.

A specific one example of use embodiment of the mononuclear cell separation apparatus 900 provided with the aforementioned constitution of the present embodiment is explained below.

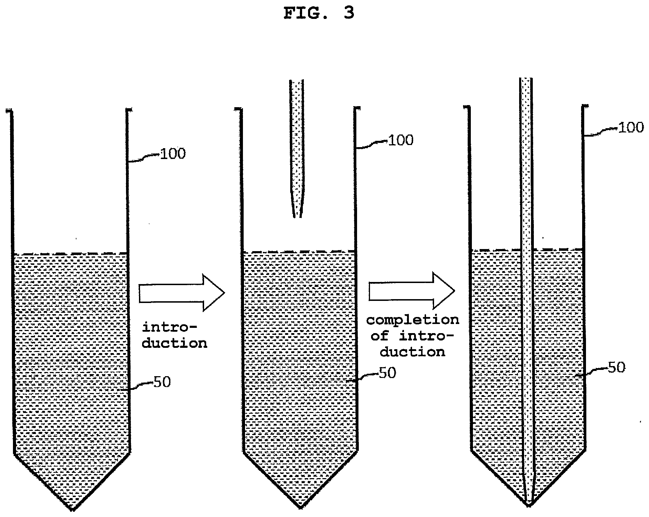

As shown in Fig. 3, left side, blood sample 50 collected from a test subject is stored in the container 100. As shown in Fig. 3, center, an opening end of a nozzle capable of injecting a centrifugation medium is introduced into the container 100 storing the blood sample. As shown in Fig. 3, right side, the opening end of the nozzle is moved to the bottom surface of the container 100 storing the blood sample.

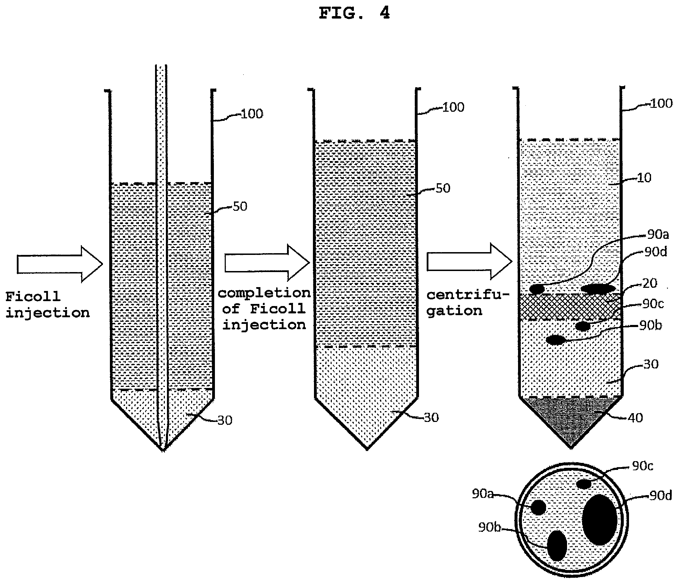

As shown in Fig. 4, left side, injection of the centrifugation medium 30 is started by the injecting means 210 and, as shown in Fig. 4, center, the centrifugation medium 30 is stored on the bottom surface side of the container 100. Thereafter, as shown in Fig. 4, right side, after centrifugation, an erythrocyte layer 40, a centrifugation medium 30, a mononuclear cell layer 20, and a plasma layer 10 are formed in this order from the container bottom part in the container 100. Furthermore, foreign object clots 90a, 90b, 90c, 90d may also be present.

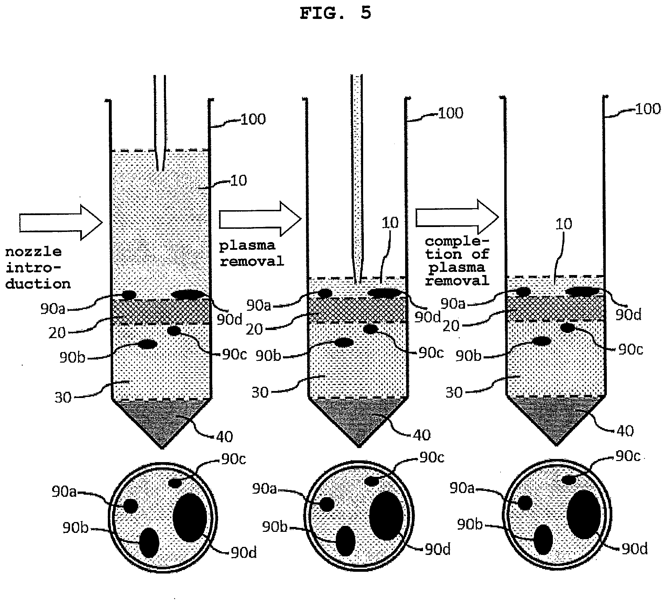

As shown in Fig. 5, left side, suction of the plasma layer 10 from the nozzle is started. Here, the nozzle is positioned above the plasma layer 10. That is, the nozzle starts removing the plasma layer 10 at a position apart from the boundary surface between the plasma layer 10 and the mononuclear cell layer 20. When a nozzle opening end at the tip of the nozzle is close to the boundary surface, the bone marrow mononuclear cells are caught in the flow generated when the plasma layer 10 is removed by suction and the yield may reduce.

As shown in Fig. 5, center, suction of the plasma layer 10 is continued until the remaining amount is small, and thereafter, as shown in Fig. 5, right side, suction of the plasma layer is completed leaving a small amount of plasma layer 10. While it is possible to entirely remove the plasma layer 10, it is preferable to complete suction of the plasma layer leaving a small amount of plasma layer 10 because the mononuclear cells present in the mononuclear cell layer 20 may also be removed by suction when removing the plasma layer 10.

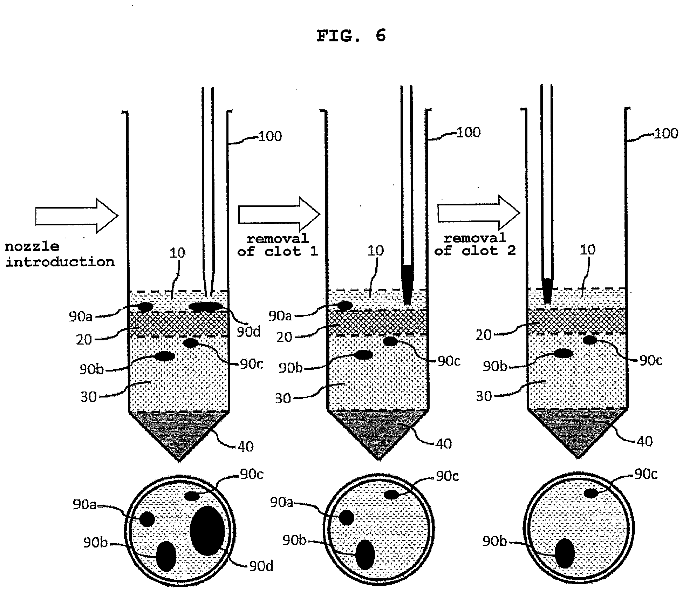

As shown in Fig. 6, left side, in this embodiment, removal of the clot 90a and 90d positioned above the mononuclear cell layer 20 is started. As shown in Fig. 6, center, clot 90d is removed by the removing means 220, and then clot 90a is removed as shown in Fig. 6, right side.

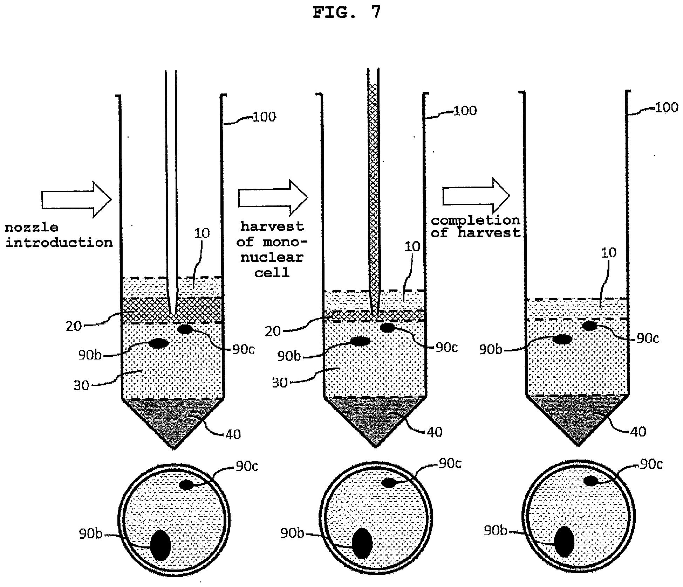

As shown in Fig. 7, left side, an opening end of a nozzle capable of harvesting mononuclear cell is introduced into the mononuclear cell layer 20 and, as shown in Fig. 7, center, suction of the mononuclear cell layer 20 from the nozzle is started and, as shown in Fig. 7, right side, harvesting of the whole mononuclear cell layer 20 is completed.

### [Example]

### (Example 1) Ischemic injury suppressive effect of bone marrow mononuclear cell

To verify an ischemic injury suppressive effect by bone marrow mononuclear cells separated by the mononuclear cell separation method of the present Example that suppressed contamination by clot, an experiment was performed using an ischemia model mouse with high reproducibility developed by the inventors (Taguchi et al. J Exp Stroke Transl Med. 2010; 3:28-33). After 48 hr from cerebral infarction, isolated bone marrow mononuclear cells (1x10⁵) were administered from the tail vein, and brain atrophy score by macroscopic specimen (Taguchi et al. Eur J Neurosci. 2007; 26(1):126-133.) was used as an evaluation method for ischemic injury suppressive effect 30 days after cell administration. In a non-treatment control group, the same amount (100 µl) of saline as in the cell administration group was administered from the tail vein. Six ischemic mice in each group were used in the experiment.

An ischemia model to verify an action of bone marrow cell administration on the microvessels was prepared by the following method.

8-Week-old SCID mouse (severe combined immunodeficiency mouse) was fully anesthetized using halothane, and the basicranium was drilled by about 1.5 mm so that the left middle cerebral artery could be directly reached by approaching from the left zygomatic region. The left middle cerebral artery immediately after passing through the olfactory tract (distal side of the olfactory crossing section) was coagulated using a bipolar cautery knife and cut after coagulation, thus permanently occluding the left middle cerebral artery, whereby an ischemia model with superior reproducibility of the ischemic site and ischemic intensity localized to the cortex of the left middle cerebral artery region was prepared.

Then, human bone marrow fluid was purchased, Ficoll-Paque PREMIUM (Ficoll is registered trademark) fluid was injected from the bottom surface of a tube (container) containing the bone marrow cell, and the tube was centrifuged at 400Xg for 40 min. Clots with major axis of 5 mm or more present above the mononuclear cell layer were removed, and mononuclear cell layer components were harvested by suction and transferred to a new tube. The cells were washed twice with albumin-containing saline (final albumin concentration 2%) by a centrifugal separator before administration.

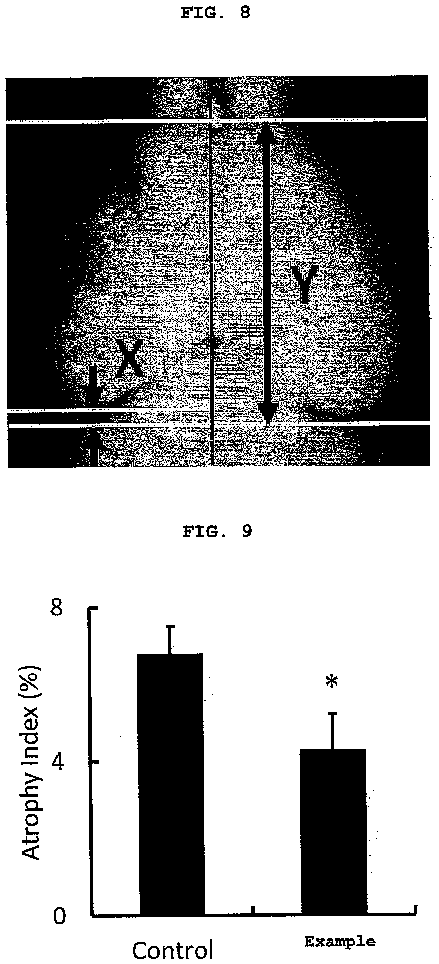

The detail of the ischemic injury score by macroscopic specimen is as follows. The cerebral ischemia model used in this study was one in which ischemia was localized to the cerebral cortex with left middle cerebral artery perfusion. After ischemia, clear tissue atrophy is developed on the ischemia side due to the infiltration of inflammatory cells and the like compared with the contralateral side (side free of cerebral ischemia). Usefulness of atrophy index for the quantitative evaluation of the level of atrophy has been shown (Taguchi et al. Eur J Neurosci. 2007; 26 (1) : 126-133.). The measurement method of atrophy index is shown in Fig. 8. The atrophy index is shown by X/Y x 100(%).

A treatment effect relating to the reduction of ischemic injury is shown in Fig. 9. Atrophy index is an index showing brain atrophy. Control is a group administered with saline. Example is a group administered with bone marrow mononuclear cells. It was shown that the administration of 1x10⁵ bone marrow mononuclear cells provides a statistically significant ischemic injury reduction effect compared with the saline administration group. "*" indicates the presence of a statistically significant difference between the Control group and the bone marrow mononuclear cell group.

From the above results, a superior ischemic injury suppressive effect of bone marrow mononuclear cell separated by the method of the present Example has been clarified.

### (Example 2) Functional recovery promoting effect from ischemic injury by bone marrow mononuclear cell

To verify a functional recovery promoting effect from ischemic injury by bone marrow mononuclear cells separated by the mononuclear cell separation method of the present Example that suppressed contamination by clot, an ischemia model mouse with high reproducibility developed by the inventors (Taguchi et al. J Exp Stroke Transl Med. 2010; 3:28-33) was used. After 48 hr from cerebral infarction, isolated bone marrow mononuclear cells (1x10⁵) were administered from the tail vein, and reactivity to light-dark conditions by open-field test (Taguchi et al. J Clin Invest. 2004; 114:330-338) was used as an evaluation method for functional recovery promoting effect from ischemic injury 30 days after cell administration. Six ischemic mice in each group were used in the experiment. The same method as in Example 1 was used for the ischemia model to verify the functional recovery promoting effect of the bone marrow cell administration. The cells to be administered were treated by the same method as in Example 1.

As an evaluation method relating to a functional recovery promoting effect, an open-field test was performed. The measurement principle and the measurement method thereof are as follows. Since mice are nocturnal, it is known that, in normal mice, the amount of activity decreases in the light condition and the amount of activity increases by darkening the environment. It has been shown that the cerebral cortex function is important for behavioral inhibition under light conditions, cerebral cortex function is recovered by cell therapy, and behavioral inhibition function under light conditions is recovered (Taguchi et al. J Clin Invest. 2004; 114:330-338). In this study, the number of standing-up actions (rearing) of mouse in an open-field measurement apparatus (manufactured by KOUIKEN, free migration space 40 cm x 40 cm) under light conditions was automatically counted for 30 min, and thereafter the number of standing-up actions under dark conditions was counted for 30 min. An increase in the quantity of motion when light conditions changed to dark conditions was used as an index of functional recovery.

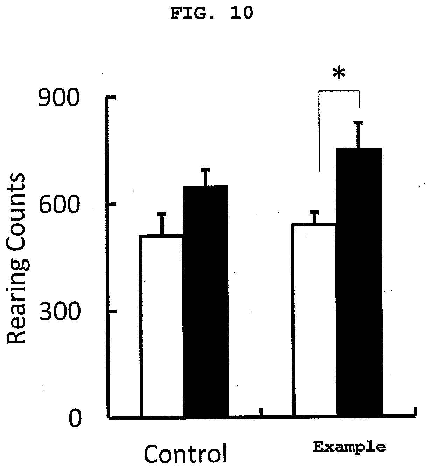

The results of the effect of promoting functional recovery from ischemic injury by the administration of bone marrow mononuclear cell are shown in Fig. 10. Control is a group administered with saline. Example is a group administered with bone marrow mononuclear cells. In Fig. 10, the number of rearing (standing-up) is shown for each of when saline was administered and when bone marrow mononuclear cell was administered, in which white shows the number of standing-up under light conditions and black shows the number of standing-up under dark conditions. In the group to which saline was administered, no significant increase in the quantity of motion was observed due to the change to the dark conditions. However, administration of bone marrow mononuclear cell resulted in the observation of a statistically significant increase in the quantity of motion relative to the dark condition. "*" shows that a statistically significant increase in the number of standing-up was found in bone marrow mononuclear cell group due to the change from the light conditions to the dark conditions. That is, it was shown that the administration of bone marrow mononuclear cell has a brain nerve functional recovery promoting effect.

From the above results, a superior cerebral cortex functional recovery promoting effect of the administration of bone marrow mononuclear cell separated by the method of the present Example has been clarified.

### [Industrial Applicability]

The present invention can be utilized for separation of bone marrow mononuclear cells.

This application is based on a patent application No. 2017-012742 filed in Japan (filing date: January 27, 2017), the contents of which are incorporated in full herein.

### [Explanation of Symbols]

- 10: plasma layer
- 20: mononuclear cell layer
- 30: centrifugation medium
- 40: erythrocyte layer
- 50: blood sample
- 90: clot
- 100: container
- 210: injecting means
- 220: removing means
- 230: harvesting means
- 300: centrifugation means
- 400: detecting means
- 410: upper imaging means
- 420: side imaging means
- 900: mononuclear cell separation apparatus

## Claims

1. A mononuclear cell separation apparatus comprising:
an injecting means (210) for injecting a centrifugation medium from the bottom surface of a container (100) storing a blood sample;
a centrifugation means (300) for centrifuging a container (100) containing a centrifugation medium and a blood sample layered in this order from the bottom surface side;
a detecting means (400) for detecting a clot present at a mononuclear cell layer after centrifugation;
a removing means (220) for removing a detected clot; and
a harvesting means (230) for harvesting the mononuclear cell.

2. The mononuclear cell separation apparatus according to claim 1, wherein the detecting means (400) comprises an upper imaging means (410) for imaging the mononuclear cell layer in the container (100) from a vertically upper direction and a side imaging means (420) for imaging the mononuclear cell layer in the container (100) from a horizontal direction.

3. The mononuclear cell separation apparatus according to claim 1 or 2, wherein the detecting means (400) detects a clot having a shape approximating a spheroid and having either an equatorial radius or a polar radius of not less than 5 mm as a clot to be a removal target.

4. The mononuclear cell separation apparatus according to any one of claims 1 to 3, comprising a plasma layer removing means for removing a plasma layer above the mononuclear cell layer after centrifugation and before removing the clot by the removing means (220).

5. The mononuclear cell separation apparatus according to claim 4, wherein the plasma layer removing means removes the plasma layer by suction from the upper part of the plasma layer.

6. The mononuclear cell separation apparatus according to claim 4 or 5, wherein the plasma layer removing means removes the plasma layer by suction while leaving 10 - 20% of a liquid amount of the plasma layer.

7. The mononuclear cell separation apparatus according to any one of claims 1 to 6, wherein the injecting means (210) injects the centrifugation medium at 0.05 - 1.0 mL/sec.

8. A method for separating a mononuclear cell, comprising:
an injecting step for injecting a centrifugation medium to the bottom surface of a container storing a blood sample;
a centrifugation step for centrifuging a container containing a centrifugation medium and a blood sample layered in this order from the bottom surface side;
a detecting step for detecting a clot present at a mononuclear cell layer after centrifugation;
a removing step for removing a detected clot; and
a harvesting step for harvesting a mononuclear cell.

9. The mononuclear cell separation method according to claim 8, wherein a clot present above the mononuclear cell layer is removed in the removing step.
